# EUROPEAN PATENT SPECIFICATION

(11) **EP 4 079 863 B1**
(45) Date of publication and mention of the grant of the patent: **24.07.2024**
(21) Application number: 21796069.9
(22) Date of filing: 20.02.2021
(51) Int. Cl.: C12Q 1/24, G01N 1/28, A01N 1/02, G01N 1/30

(54) **CELL INSPECTION METHOD USING JELLIFICATION OF ALCOHOL-BASED SOLUTION COMPOSITION**
VERFAHREN ZUR ZELLINSPEKTION MITTELS GELIERUNG EINER LÖSUNGSZUSAMMENSETZUNG AUF ALKOHOLBASIS
PROCÉDÉ D'INSPECTION DE CELLULE UTILISANT LA GÉLIFICATION D'UNE COMPOSITION DE SOLUTION À BASE D'ALCOOL

(30) Priority: 29.04.2020 KR 20200052928
(43) Date of publication of application: 26.10.2022
(73) Proprietor: Biodyne Co., Ltd., Seongdong-gu Seoul 04793 (KR)
(72) Inventor: IM, Wook Bin, Gangnam-gu, Seoul 06280 (KR)
(74) Representative: Hoefer & Partner Patentanwälte mbB
(86) International application number: PCT/KR2021/002160
(87) International publication number: WO 2021/221280

(56) References cited:
- EP-B1- 0 511 430
- WO-A1-2011/146909
- WO-A1-2020/020913
- WO-A2-2017/083729
- JP-A- 2017 521 642
- KR-A- 20050 116 689
- KR-B1- 100 849 035
- KR-B1- 102 212 669
- US-A- 6 051 425
- US-A1- 2003 104 347
- US-A1- 2005 074 422
- US-A1- 2016 327 457

## Description

### Field of the Invention

The present invention relates to a technology that jellifies a specimen (for example, exfoliative cells of a human body) in a vial, safely transports the specimen to an examination center, restores the specimen to its original state (for example, conversion into liquid phase) in the examination center, and conducts examination of the specimen.

More specifically, the present invention relates to a technology that converts a liquid for specimen preservation contained in a vial into a jelly state, safely transports the specimen to an examination center without deformation, redissolves the liquid for specimen preservation into a liquid phase at the examination center, and conducts examination of the specimen.

### Background Art

In general, the state of exfoliative cells can be observed for diagnosis after a specimen (exfoliative cells) is obtained from the human body and then the exfoliative cells are spread on the slide for cytodiagnosis at the examination center.

Here, patients who have difficulty visiting the examination center should collect their own exfoliative cells (specimen) and then have the exfoliative cells transported to the examination center in a state in which the exfoliative cells are intact. To this end, the specimen may be put in a vial, which is an airtight container, together with the liquid for cell fixation to protect the specimen and transported to the examination center.

The liquid for cell fixation filled in the vial to prevent specimen deformation is generally composed of an aqueous solution. In the process of transporting the vial, the liquid for cell fixation frequently leaks through the gap between the body and the cap of the vial, and this may cause deformation of the specimen.

It is required to develop a technology capable of solving such problems of the prior art.

Meanwhile, the prior literatures related to the present invention are as follows.
(1) EP 0511430 A2 (November 04, 1992) "Cell preservative solution"
(2) US 2006/0088814 A1 (April 27, 2006) "Enhanced cell preservative solution and methods for using same"
(3) Korean Patent Application Laid-Open No. 10-2005-0116689 (December 13, 2005) "Gel preservative, method for preparing the same, and cell examining method using the same"

### DISCLOSURE OF INVENTION

### Technical Problem

The present invention has been proposed in view of the above points, and an object thereof is to provide a cell examining method using jellification of an alcohol-based solution composition, by which a specimen (for example, exfoliative cells of a human body) contained in a vial is jellified with an alcohol-based solution composition so that the specimen can be safely transported over a long distance and is easily restored to its original state when cell examination is conducted at an examination center.

### Technical Solution

In order to achieve the object, the cell examining method using jellification of an alcohol-based solution composition according to the first embodiment of the present invention may be configured to include (a) mixing any one solution selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive with any one material selected from 5 to 20 parts by weight of one or more of gelatin or a photocurable protein, 1 to 100 parts by weight of a natural cellulose polymer material, or 1 to 100 parts by weight of synthesized acrylic polymer material beads at a temperature of 1°C to 40°C for jellification to produce an alcohol-based jelly-like material; (b) putting a collected specimen inside an airtight container containing the alcohol-based jelly-like material; (d) adding the jellified alcohol-based jelly-like material in the airtight container to a solution for redissolution; (e) maintaining the solution for redissolution at a temperature of 1°C to 40°C to redissolve the alcohol-based jelly-like material into a liquid phase; and (f) obtaining the specimen from the alcohol-based jelly-like material in a redissolved state and examining the specimen.

The cell examining method using jellification of an alcohol-based solution composition according to the second embodiment of the present invention may be configured to include (a) putting any one solution (hereinafter referred to as 'selected solution') selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive into an airtight container; (b) putting a specimen inside the airtight container filled with the selected solution; (c) putting any one material selected from 5 to 20 parts by weight of one or more of gelatin or a photocurable protein, 1 to 100 parts by weight of a natural cellulose polymer material, or 1 to 100 parts by weight of synthesized acrylic polymer material beads inside the airtight container in a state of containing the selected solution and the specimen at a temperature of 1°C to 40°C and jellifying the mixture into an alcohol-based jelly-like material; (d) adding the jellified alcohol-based jelly-like material in the airtight container to a solution for redissolution; (e) maintaining the solution for redissolution at a temperature of 1°C to 40°C to redissolve the jellified alcohol-based jelly-like material into a liquid material; and (f) obtaining the specimen from the liquid material in a redissolved state and examining the specimen.

At this time, step (d) may include a step of preparing a solution for redissolution configured to contain 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution.

In addition, step (d) may include a step of preparing a solution for redissolution configured to contain 40 to 50 parts by weight of an aqueous ethanol solution, 0.1 to 0.2 parts by weight of EDTA, and 0.2 to 1 part by weight of a dihydric alcohol additive.

### Advantageous Effects

The present invention has an advantage that the jellification of a liquid for cell fixation filled inside a vial is simple since the liquid for cell fixation is jellified by mixing the selected solution and the selected material.

The present invention has an advantage that the leakage of a liquid for cell fixation from a vial can be prevented during long-distance transport by the jellification of the liquid for cell fixation.

The present invention also has an advantage that conversion from a jellified state into a liquid phase for cell examination is simple.

### Brief Description of the Drawings

FIG. 1 is an exemplary view of a vial, which is an airtight container that contains a specimen for transport.
FIG. 2 is an exemplary view of a state in which a vial is mounted on a stirrer for jellification.
FIG. 3 is an exemplary view of a state in which a vial is mounted on a stirrer for redissolution.
FIG. 4 is a flow chart illustrating a cell examining process using jellification of an alcohol-based solution composition according to a first embodiment of the present invention.
FIG. 5 is a flow chart illustrating a cell examining process using jellification of an alcohol-based solution composition according to a second embodiment of the present invention.

### Embodiment for Carrying Out the Invention

Hereinafter, the present invention will be described in detail.

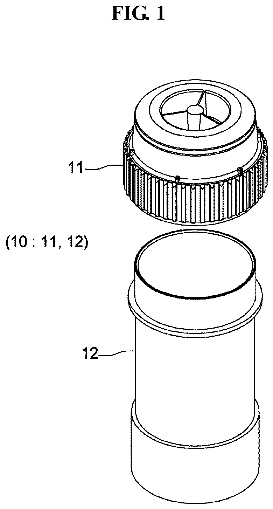

FIG. 1 is an exemplary view of a vial, which is an airtight container that contains a specimen for transport. Referring to FIG. 1, a vial 10 may include a hollow body 12 having open one end and a cap 11 opening and closing the opening portion of the body 12.

At this time, the vial 10 may be fabricated by extruding a resin containing an antioxidant since the alcohol component may be oxidized when the vial 10 is exposed to light or oxygen in a state of containing the liquid for cell fixation. The vial 10 may also be fabricated to be translucent by adding a light blocking dye.

It may be possible to prevent the exposure to oxygen or light by adopting an opaque pouch-type packaging material as the container containing the liquid for cell fixation.

Meanwhile, the specimen portion not submerged in the liquid for cell fixation may be damaged in the process of putting the specimen-collected brush tip (not illustrated) inside the vial 10. It is thus preferable that the vial 10 is fabricated in a size that can completely accommodate the brush tip. It is also preferable to fully fill the liquid for cell fixation in the vial 10 in a state in which the specimen-collected brush tip is placed inside the vial 10.

Conventionally, when the liquid for cell fixation in the form of an aqueous solution was filled in the vial 10 together with the specimen, a problem of specimen deformation due to leakage during long-distance transport has occurred. In order to solve this problem, the present invention provides a composition capable of jellifying the liquid for cell fixation. In other words, when the liquid for cell fixation is jellified, the possibility of leakage or evaporation is low, and the original state of specimen can be maintained.

Here, the liquid for cell fixation contained in the vial 10 is transported to the examination center in a jelly state, and the jellified state is maintained by maintaining the vial 10 in the low temperature state of room temperature or below during the transporting process.

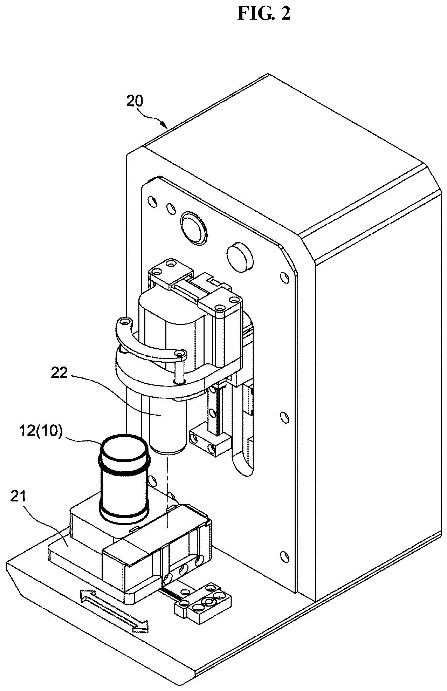

FIG. 2 is an exemplary view of a state in which the vial 10 is mounted on a stirrer for jellification 20. Referring to FIG. 2, the stirrer for jellification 20 may be equipped as a means for mixing the selected solution and the selected material according to the present invention to produce an alcohol-based jelly-like material.

First, the vial 10 is mounted on a table member 21 of the stirrer for jellification 20. In this state, the stirrer for jellification 20 is operated to position the table member 21 under an injection member 22, and the jelly composition is filled in the vial 10 as the jelly composition is dropped downward from the injection member 22.

The jelly composition in the vial 10 may be thoroughly mixed by operating the stirrer for jellification 20 and thus reciprocating the table member 21 in the direction of the arrow in FIG. 2.

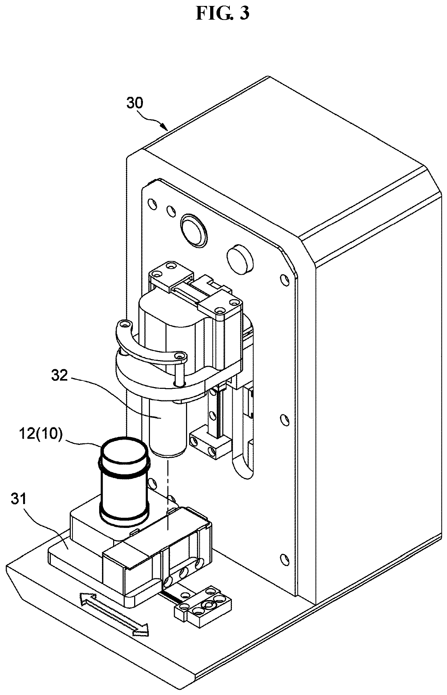

FIG. 3 is an exemplary view of a state in which the vial 10 is mounted on a stirrer for redissolution 30. When the alcohol-based jelly-like material according to the present invention arrives at the examination center in a state of being contained in the vial 10 together with the specimen, the alcohol-based jelly-like material in the vial 10 is required to be reconverted into a liquid phase before the specimen is taken out from the vial 10.

To this end, the vial 10 is mounted on a table member 31 of the stirrer for redissolution 30. In this state, the stirrer for redissolution 30 is operated to position the table member 31 under an injection member 32, and the alcohol-based jelly-like material in the vial 10 is reconverted into a liquid phase as the solution for redissolution is dropped downward from the injection member 32.

At this time, the solution for redissolution in the vial 10 may be thoroughly mixed with the jelly composition by operating the stirrer for redissolution 30 and thus reciprocating the table member 31 in the direction of the arrow in FIG. 3.

In the present specification, the stirrer for jellification 20 of FIG. 2 and the stirrer for redissolution 30 of FIG. 3 have been described with different names for convenience of explanation, but the same apparatus may be used. The stirrer for jellification 20 of FIG. 2 and the stirrer for redissolution 30 of FIG. 3 have been illustrated in similar forms, but may be configured in different forms.

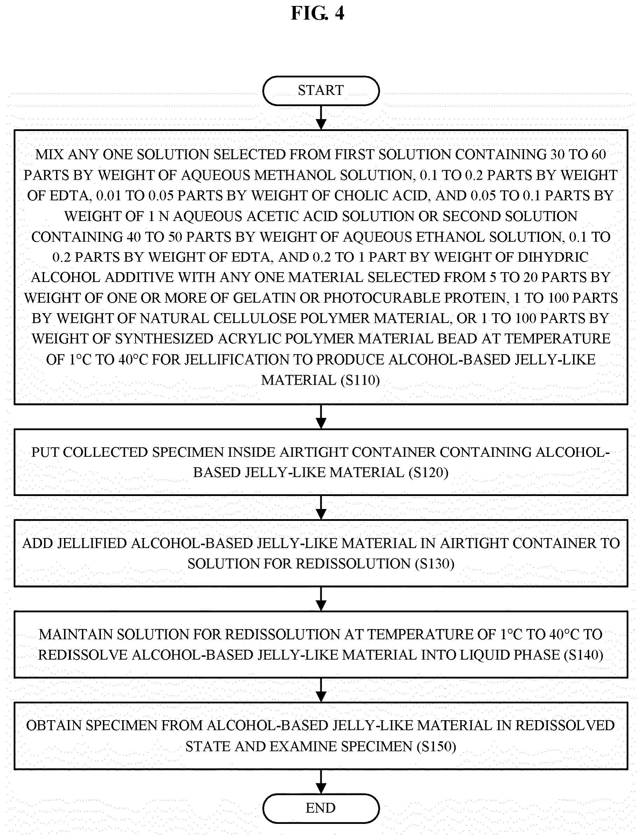

FIG. 4 is a flow chart illustrating a cell examining process using jellification of an alcohol-based solution composition according to the first embodiment of the present invention.

Step S 110: First, any one solution (hereinafter referred to as 'selected solution') is selected from the first solution containing 30 to 60 parts by weight of an aqueous methanol solution, 0.1 to 0.2 parts by weight of EDTA (ethylenediaminetetraacetic acid), 0.01 to 0.05 parts by weight of cholic acid, and 0.05 to 0.1 parts by weight of a 1 N aqueous acetic acid solution or the second solution containing 40 to 50 parts by weight of an aqueous ethanol solution, 0.1 to 0.2 parts by weight of EDTA, and 0.2 to 1 part by weight of a dihydric alcohol additive.

In addition, any one material (hereinafter referred to as 'selected material') is selected from 5 to 20 parts by weight of one or more of gelatin or a photocurable protein (gelatin methacryloly; GelMA), 1 to 100 parts by weight of a natural cellulose polymer material, or 1 to 100 parts by weight of synthesized acrylic polymer material beads.

Subsequently, the selected solution and the selected material are put into the vial 10 and then mixed at a temperature of 1°C to 40°C for jellification to produce an alcohol-based jelly-like material.

At this time, when the temperature around the vial 10 is as low as less than 1°C, the gelatin, photocurable protein, natural cellulose polymer material, and synthesized acrylic polymer beads in the vial 10 do not dissolve, resulting in poor gelation or jellification.

When the temperature around the vial 10 is as high as more than 40°C, the alcohol-based components having low boiling points evaporate, thus changes in the physical properties of the entire composition are caused, and the specimen fixing ability of the cell fixing means is too strong or weak.

Step S120: Next, a specimen is put inside the vial 10 containing the alcohol-based jelly-like material. At this time, it is preferable that the vial 10 is fabricated in a size that can completely accommodate the specimen-collected brush tip (not illustrated) so that the brush tip is safely placed inside the vial 10.

The vial 10 in the state of containing the specimen in this way is transported to the examination center. During the transporting process, the vial 10 is required to be maintained in a low temperature state of room temperature or below to prevent the conversion of the jelly state into a liquid phase.

When the vial 10 in the state of containing the specimen is transported, the specimen is required to be taken out from the vial 10 and subjected to the cell examination at the examination center. At this time, in order to safely take out the specimen from the vial 10 without deformation, the cell fixing means in the vial 10 is required to be reconverted into a liquid phase.

Steps S130 and S140: The jellified alcohol-based jelly-like material in the vial 10 may be redissolved into a liquid phase by adding the alcohol-based jelly-like material to the solution for redissolution and then maintaining the solution for redissolution at a temperature of 1°C to 40°C.

At this time, an embodiment is possible in which the solution for redissolution is dropped inside the vial 10 while the vial 10 containing the jelly-like material and the specimen is mounted on the mechanism of the stirrer for redissolution 30 as in FIG. 3. An embodiment is also possible in which the vial 10 is placed in the solution for redissolution separately from the stirrer for redissolution 30.

Meanwhile, the solution for redissolution in the first embodiment of the present invention may be configured to contain 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution.

The solution for redissolution in the first embodiment of the present invention may also be configured to contain 40 to 50 parts by weight of an aqueous ethanol solution, 0.1 to 0.2 parts by weight of EDTA, and 0.2 to 1 part by weight of a dihydric alcohol additive.

Step S150: As in FIG. 3, after the material in the vial 10 is reconverted into a liquid phase, the specimen in the vial 10 may be taken out using a separate apparatus (not illustrated) and subjected to the cell examination.

In the first embodiment of the present invention, the selected solution is selected from the first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or the second solution containing 40 to 50 parts by weight of an aqueous ethanol solution, 0.1 to 0.2 parts by weight of EDTA, and 0.2 to 1 part by weight of a dihydric alcohol additive. The selected material is selected from 5 to 20 parts by weight of one or more of gelatin or a photocurable protein, 1 to 100 parts by weight of a natural cellulose polymer material, or 1 to 100 parts by weight of synthesized acrylic polymer material beads.

The physical properties depending on the composition ratio were examined for the first embodiment of the present invention as described above, and the influence of an individual component on the physical properties of the entire composition was examined while varying the mixing ratio of the individual component.

Table 1 presents the experimental conditions in which the mixing ratio of only the aqueous methanol solution is varied while the mixing ratios of other components are fixed in the first embodiment of the present invention.

**[Table 1]**

| "Experiment 1" | Example | | | Comparative Example | |
|---|---|---|---|---|---|
| | A1 | B1 | C1 | a1 | b1 |
| Aqueous methanol solution (30-60) | 30 | 45 | 60 | 25 | 65 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 2 presents the experimental conditions in which the mixing ratio of only EDTA is varied while the mixing ratios of other components are fixed in the first embodiment of the present invention.

**[Table 2]**

| "Experiment 2" | Example | | | Comparative Example | |
|---|---|---|---|---|---|
| | A2 | B2 | C2 | a2 | b2 |
| Aqueous methanol solution (30-60) | 45 | 45 | 45 | 45 | 45 |
| EDTA (0.1-0.2) | 0.1 | 0.15 | 0.2 | 0.05 | 0.25 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 3 presents the experimental conditions in which the mixing ratio of only cholic acid is varied while the mixing ratios of other components are fixed in the first embodiment of the present invention.

**[Table 3]**

| "Experiment 3" | Example | | | Comparative Example | |
|---|---|---|---|---|---|
| | A3 | B3 | C3 | a3 | b3 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.02 | 0.03 | 0.04 | 0.00 | 0.07 |
| 1 N aqueous acetic acid solution (0.05-0.1) | 0.07 | 0.07 | 0.07 | 0.07 | 0.07 |
| Gelatin (5-20) | 13 | 13 | 13 | 13 | 13 |

Table 4 presents the experimental conditions in which the mixing ratio of only the 1 N aqueous acetic acid solution is varied while the mixing ratios of other components are fixed in the first embodiment of the present invention.

**[Table 4]**

| "Experiment 4" | Example | | | Comparative Example | |
|---|---|---|---|---|---|
| | A4 | B4 | C4 | a4 | b4 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.06 | 0.07 | 0.09 | 0.03 | 0.13 |
| Gelatin (5-20) | 13 | 13 | 13 | 13 | 13 |

Table 5 presents the experimental conditions in which the mixing ratio of only gelatin is varied while the mixing ratios of other components are fixed in the first embodiment of the present invention.

**[Table 5]**

| "Experiment 5" | Example | | | Comparative Example | |
|---|---|---|---|---|---|
| | A5 | B5 | C5 | a5 | b5 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |
| Gelatin (5-20) | 6 | 13 | 19 | 3 | 23 |

In Tables 1 to 5, in Examples (A1 to A5, B1 to B5, and C1 to C5), the fixing ability of the jellified cell fixing means was favorable. On the other hand, in Comparative Examples (a1 to a5 and b1 to b5), the ability to fix the specimen inside the vial 10 was too strong or weak. Therefore, in Comparative Examples, a problem (for example, specimen deformation) occurred in the process (for example, redissolution) of taking out the specimen from the vial 10.

With regard to the first embodiment of the present invention, experiments were conducted in addition to those presented in Tables 1 to 5, but the description of other experiments has been omitted for convenience of explanation.

Meanwhile, in the alcohol-based jelly-like material according to the first embodiment of the present invention, 5 to 20 parts by weight of any one or more of gelatin or a photocurable protein may be substituted with 1 to 100 parts by weight of a natural cellulose polymer (for example, mecellose or hecellose) or 1 to 100 parts by weight of synthesized acrylic polymer beads (for example, methyl methacrylate-co-ethylene glycol dimethacrylate; MMA).

Here, with regard to the natural cellulose polymer as well, an experiment was conducted in mixing ratio ranges of the natural cellulose polymer of less than 1 part by weight and more than 100 parts by weight as Comparative Examples while the mixing ratios of other components were fixed, and as a result, the ability to fix the specimen inside the vial 10 was not favorable in the region outside of 1 to 100 parts by weight.

In addition, with regard to the synthesized acrylic polymer beads as well, an experiment was conducted in mixing ratio ranges of the synthesized acrylic polymer beads of less than 1 part by weight and more than 100 parts by weight as Comparative Examples while the mixing ratios of other components were fixed, and as a result, the ability to fix the specimen inside the vial 10 was not favorable in the region outside of 1 to 100 parts by weight.

Meanwhile, the solution for redissolution used in step S130 in the first embodiment of the present invention may be configured to contain 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution (hereinafter referred to as 'first solution for redissolution').

The physical properties depending on the composition ratio of such a first solution for redissolution were examined in the first embodiment of the present invention, and the influence of an individual component on the physical properties of the entire composition was examined while varying the mixing ratio of the individual component.

Table 6 presents the experimental conditions in which the mixing ratio of only the aqueous methanol solution is varied while the mixing ratios of other components are fixed in the redissolving process using the first solution for redissolution in the first embodiment of the present invention.

**[Table 6]**

| "Experiment 6" | Example | | | Comparative Example | |
|---|---|---|---|---|---|
| | A6 | B6 | C6 | a6 | b6 |
| Aqueous methanol solution (30-60) | 30 | 45 | 60 | 25 | 65 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |

Table 7 presents the experimental conditions in which the mixing ratio of only EDTA is varied while the mixing ratios of the components are fixed in the redissolving process using the first solution for redissolution in the first embodiment of the present invention.

**[Table 7]**

| "Experiment 7" | Example | | | Comparative Example | |
|---|---|---|---|---|---|
| | A7 | B7 | C7 | a7 | b7 |
| Aqueous methanol solution (30-60) | 45 | 45 | 45 | 45 | 45 |
| EDTA (0.1-0.2) | 0.1 | 0.15 | 0.2 | 0.05 | 0.25 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |

Table 8 presents the experimental conditions in which the mixing ratio of only cholic acid is varied while the mixing ratios of other components are fixed in the redissolving process using the first solution for redissolution in the first embodiment of the present invention.

**[Table 8]**

| "Experiment 8" | Example | | | Comparative Example | |
|---|---|---|---|---|---|
| | A8 | B8 | C8 | a8 | b8 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.02 | 0.03 | 0.04 | 0.00 | 0.07 |
| 1 N aqueous acetic acid solution (0.05-0.1) | 0.07 | 0.07 | 0.07 | 0.07 | 0.07 |

Table 9 presents the experimental conditions in which the mixing ratio of only the 1 N aqueous acetic acid solution is varied while the mixing ratios of other components are fixed in the redissolving process using the first solution for redissolution in the first embodiment of the present invention.

**[Table 9]**

| "Experiment 9" | Example | | | Comparative Example | |
|---|---|---|---|---|---|
| | A9 | B9 | C9 | a9 | b9 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.06 | 0.07 | 0.09 | 0.03 | 0.13 |

In Tables 6 to 9, in Examples (A6 to A9, B6 to B9, and C6 to C9), there was no particular problem in the process (for example, redissolution) of taking out the specimen from the vial 10. On the other hand, in Comparative Examples (a6 to a9 and b6 to b9), a problem (for example, specimen deformation) occurred in the process (for example, redissolution) of taking out the specimen from the vial 10.

Meanwhile, the solution for redissolution in the first embodiment of the present invention may be configured to contain 40 to 50 parts by weight of an aqueous ethanol solution, 0.1 to 0.2 parts by weight of EDTA, and 0.2 to 1 part by weight of a dihydric alcohol additive (hereinafter referred to as 'second solution for redissolution').

The physical properties depending on the composition ratio of such a second solution for redissolution were examined in the first embodiment of the present invention, and the influence of an individual component on the physical properties of the entire composition was examined while varying the mixing ratio of the individual component.

Table 10 presents the experimental conditions in which the mixing ratio of only the aqueous ethanol solution is varied while the mixing ratios of other components are fixed in the redissolving process using the second solution for redissolution in the first embodiment of the present invention.

**[Table 10]**

| "Experiment 10" | Example | | | Comparative Example | |
|---|---|---|---|---|---|
| | A10 | B10 | C10 | a10 | b10 |
| Aqueous ethanol solution (40-50) | 40 | 45 | 50 | 30 | 60 |
| EDTA (0.1-0.2) | 0.15 | 0.15 | 0.15 | 0.15 | 0.15 |
| Dihydric alcohol additive (0.2-1) | 0.6 | 0.6 | 0.6 | 0.6 | 0.6 |

Table 11 presents the experimental conditions in which the mixing ratio of only EDTA is varied while the mixing ratios of other components are fixed in the redissolving process using the second solution for redissolution in the first embodiment of the present invention.

**[Table 11]**

| "Experiment 11" | Example | | | Comparative Example | |
|---|---|---|---|---|---|
| | A11 | B11 | C11 | a11 | b11 |
| Aqueous ethanol solution (40-50) | 45 | 45 | 45 | 45 | 45 |
| EDTA (0.1-0.2) | 0.1 | 0.15 | 0.2 | 0.05 | 0.25 |
| Dihydric alcohol additive (0.2-1) | 0.6 | 0.6 | 0.6 | 0.6 | 0.6 |

Table 12 presents the experimental conditions in which the mixing ratio of only the dihydric alcohol additive is varied while the mixing ratios of other components are fixed in the redissolving process using the second solution for redissolution in the first embodiment of the present invention.

**[Table 12]**

| "Experiment 11" | Example | | | Comparative Example | |
|---|---|---|---|---|---|
| | A12 | B12 | C12 | a12 | b12 |
| Aqueous ethanol solution (40-50) | 45 | 45 | 45 | 45 | 45 |
| EDTA (0.1-0.2) | 0.15 | 0.15 | 0.15 | 0.15 | 0.15 |
| Dihydric alcohol additive (0.2-1) | 0.3 | 0.6 | 0.9 | 0.1 | 1.4 |

In Tables 10 to 12, in Examples (A10 to A12, B10 to B12, and C10 to C12), there was no particular problem in the process (for example, redissolution) of taking out the specimen from the vial 10. On the other hand, in Comparative Examples (a10 to a12 and b10 to b 12), a problem (for example, specimen deformation) occurred in the process (for example, redissolution) of taking out the specimen from the vial 10.

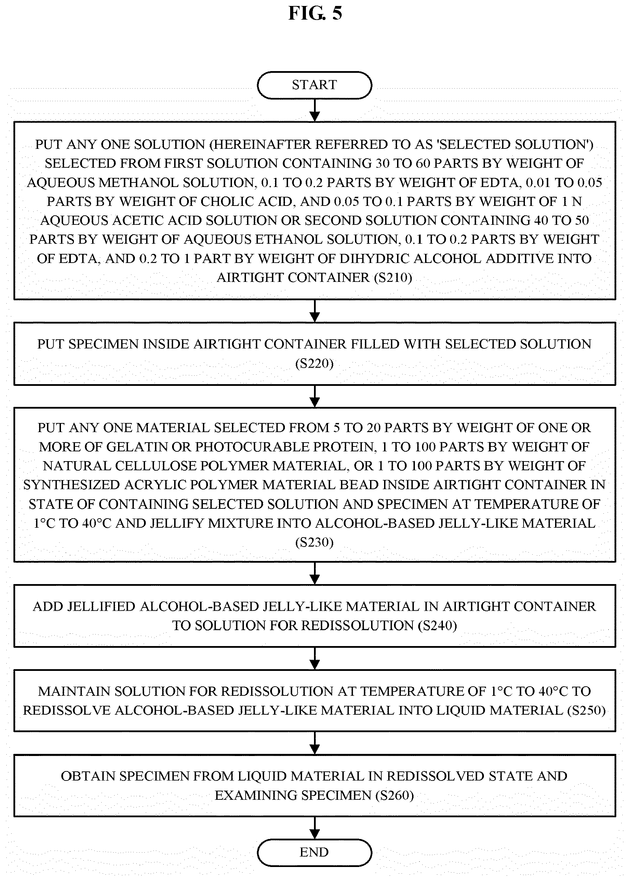

FIG. 5 is a flow chart illustrating a cell examining process using jellification of the alcohol-based solution composition according to the second embodiment of the present invention.

Step S210: First, any one solution (hereinafter referred to as 'selected solution') is selected from the first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or the second solution containing 40 to 50 parts by weight of an aqueous ethanol solution, 0.1 to 0.2 parts by weight of EDTA, and 0.2 to 1 part by weight of a dihydric alcohol additive, and then the selected solution is put into the vial 10.

Step S220: Next, a specimen is put inside the vial 10. At this time, it is preferable that the vial 10 is fabricated in a size that can completely accommodate the brush tip.

Step S230: Subsequently, any one material (hereinafter referred to as 'selected material') is selected from 5 to 20 parts by weight of one or more of gelatin or a photocurable protein, 1 to 100 parts by weight of a natural cellulose polymer material, or 1 to 100 parts by weight of synthesized acrylic polymer material beads.

Subsequently, the selected material is put inside the vial 10 in the state of containing the specimen while the temperature around the vial 10 is maintained at a temperature of 1°C to 40°C, and the mixture is jellified into an alcohol-based jelly-like material.

At this time, when the temperature around the vial 10 is as low as less than 1°C, the gelatin, photocurable protein, natural cellulose polymer material, and synthesized acrylic polymer in the vial 10 do not dissolve, resulting in poor gelation or j ellification.

When the temperature around the vial 10 is as high as more than 40°C, the alcohol-based components having low boiling points evaporate, thus changes in the physical properties of the entire composition are caused, and the specimen fixing ability of the cell fixing means is too strong or weak.

Thereafter, the vial 10 in the state of containing the specimen is transported to the examination center. During the transporting process, the vial 10 is required to be maintained in a low temperature state of room temperature or below to prevent the conversion of the jelly state into a liquid phase.

When the vial 10 in the state of containing the specimen is transported, the specimen is required to be taken out from the vial 10 and subjected to the cell examination at the examination center. At this time, in order to safely take out the specimen from the vial 10 without deformation, the cell fixing means in the vial 10 is required to be reconverted into a liquid phase.

Steps S240 and S250: The jellified alcohol-based jelly-like material in the vial 10 may be redissolved into a liquid phase by adding the alcohol-based jelly-like material to the solution for redissolution and then maintaining the solution for redissolution at a temperature of 1°C to 40°C.

At this time, an embodiment is possible in which the solution for redissolution is dropped inside the vial 10 while the vial 10 containing the jelly-like material and the specimen is mounted on the mechanism of the stirrer for redissolution 30 as in FIG. 3. An embodiment is also possible in which the vial 10 is placed in the solution for redissolution separately from the stirrer for redissolution 30.

Meanwhile, the solution for redissolution in the second embodiment of the present invention may be configured to contain 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution.

The solution for redissolution in the second embodiment of the present invention may also be configured to contain 40 to 50 parts by weight of an aqueous ethanol solution, 0.1 to 0.2 parts by weight of EDTA, and 0.2 to 1 part by weight of a dihydric alcohol additive.

Step S260: As in FIG. 3, after the material in the vial 10 is reconverted into a liquid phase, the specimen in the vial 10 may be taken out using a separate apparatus (not illustrated) and subjected to the cell examination.

In the second embodiment of the present invention, the selected solution is selected from the first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or the second solution containing 40 to 50 parts by weight of an aqueous ethanol solution, 0.1 to 0.2 parts by weight of EDTA, and 0.2 to 1 part by weight of a dihydric alcohol additive. The selected material is selected from 5 to 20 parts by weight of one or more of gelatin or a photocurable protein, 1 to 100 parts by weight of a natural cellulose polymer material, or 1 to 100 parts by weight of synthesized acrylic polymer material beads.

The physical properties depending on the composition ratio were examined for the second embodiment of the present invention as described above, and the influence of an individual component on the physical properties of the entire composition was examined while varying the mixing ratio of the individual component.

Table 13 presents the experimental conditions in which the mixing ratio of only the aqueous ethanol solution is varied while the mixing ratios of other components are fixed in the second embodiment of the present invention.

**[Table 13]**

| "Experiment 13" | Example | | | Comparative Example | |
|---|---|---|---|---|---|
| | A13 | B13 | C13 | a13 | b13 |
| Aqueous ethanol solution (40-50) | 40 | 45 | 50 | 30 | 60 |
| EDTA (0.1-0.2) | 0.15 | 0.15 | 0.15 | 0.15 | 0.15 |
| Dihydric alcohol additive (0.2-1) | 0.6 | 0.6 | 0.6 | 0.6 | 0.6 |
| Gelatin (5-20) | 13 | 13 | 13 | 13 | 13 |

Table 14 presents the experimental conditions in which the mixing ratio of only EDTA is varied while the mixing ratios of other components are fixed in the second embodiment of the present invention.

**[Table 14]**

| "Experiment 14" | Example | | | Comparative Example | |
|---|---|---|---|---|---|
| | A14 | B14 | C14 | a14 | b14 |
| Aqueous ethanol solution (40-50) | 45 | 45 | 45 | 45 | 45 |
| EDTA (0.1-0.2) | 0.1 | 0.15 | 0.2 | 0.05 | 0.25 |
| Dihydric alcohol additive (0.2-1) | 0.6 | 0.6 | 0.6 | 0.6 | 0.6 |
| Gelatin (5-20) | 13 | 13 | 13 | 13 | 13 |

Table 15 presents the experimental conditions in which the mixing ratio of only the dihydric alcohol additive is varied while the mixing ratios of other components are fixed in the second embodiment of the present invention.

**[Table 15]**

| "Experiment 15" | Example | | | Comparative Example | |
|---|---|---|---|---|---|
| | A15 | B15 | C15 | a15 | b15 |
| Aqueous ethanol solution (40-50) | 45 | 45 | 45 | 45 | 45 |
| EDTA (0.1-0.2) | 0.15 | 0.15 | 0.15 | 0.15 | 0.15 |
| Dihydric alcohol additive (0.2-1) | 0.3 | 0.6 | 0.9 | 0.1 | 1.4 |
| Gelatin (5-20) | 13 | 13 | 13 | 13 | 13 |

Table 16 presents the experimental conditions in which the mixing ratio of only gelatin is varied while the mixing ratios of other components are fixed in the second embodiment of the present invention.

**[Table 16]**

| "Experiment 16" | Example | | | Comparative Example | |
|---|---|---|---|---|---|
| | A16 | B16 | C16 | a16 | b16 |
| Aqueous ethanol solution (40-50) | 45 | 45 | 45 | 45 | 45 |
| EDTA (0.1-0.2) | 0.15 | 0.15 | 0.15 | 0.15 | 0.15 |
| Dihydric alcohol additive (0.2-1) | 0.3 | 0.6 | 0.9 | 0.1 | 1.4 |
| Gelatin (5-20) | 6 | 13 | 19 | 3 | 23 |

In Tables 13 to 16, in Examples (A13 to A16, B13 to B16, and C13 to C16), the fixing ability of the jellified cell fixing means was favorable. On the other hand, in Comparative Examples (a13 to a16 and b13 to b16), the ability to fix the specimen inside the vial 10 was too strong or weak. Therefore, in Comparative Examples, a problem (for example, specimen deformation) occurred in the process (for example, redissolution) of taking out the specimen from the vial 10.

With regard to the second embodiment of the present invention, experiments were conducted in addition to those presented in Tables 13 to 16, but the description of other experiments has been omitted for convenience of explanation.

Meanwhile, in the second embodiment of the present invention, 5 to 20 parts by weight of any one or more of gelatin or a photocurable protein may be substituted with 1 to 100 parts by weight of a natural cellulose polymer (for example, mecellose or hecellose) or 1 to 100 parts by weight of synthesized acrylic polymer beads (for example, methyl methacrylate-co-ethylene glycol dimethacrylate; MMA).

Here, with regard to the natural cellulose polymer as well, an experiment was conducted in mixing ratio ranges of the natural cellulose polymer of less than 1 part by weight and more than 100 parts by weight as Comparative Examples while the mixing ratios of other components were fixed, and as a result, the ability to fix the specimen inside the vial 10 was not favorable in the region outside of 1 to 100 parts by weight.

In addition, with regard to the synthesized acrylic polymer beads as well, an experiment was conducted in mixing ratio ranges of the synthesized acrylic polymer beads of less than 1 part by weight and more than 100 parts by weight as Comparative Examples while the mixing ratios of other components were fixed, and as a result, the ability to fix the specimen inside the vial 10 was not favorable in the region outside of 1 to 100 parts by weight.

Meanwhile, the solution for redissolution used in step S240 in the second embodiment of the present invention may be configured to contain 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution (hereinafter referred to as 'third solution for redissolution').

The physical properties depending on the composition ratio of such a third solution for redissolution were examined in the second embodiment of the present invention, and the influence of an individual component on the physical properties of the entire composition was examined while varying the mixing ratio of the individual component.

Table 17 presents the experimental conditions in which the mixing ratio of only the aqueous methanol solution is varied while the mixing ratios of other components are fixed in the redissolving process using the third solution for redissolution in the second embodiment of the present invention.

**[Table 17]**

| "Experiment 17" | Example | | | Comparative Example | |
|---|---|---|---|---|---|
| | A17 | B17 | C17 | a17 | b17 |
| Aqueous methanol solution (30-60) | 30 | 45 | 60 | 25 | 65 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |

Table 18 presents the experimental conditions in which the mixing ratio of only EDTA is varied while the mixing ratios of other components are fixed in the redissolving process using the third solution for redissolution in the second embodiment of the present invention.

**[Table 18]**

| "Experiment 18" | Example | | | Comparative Example | |
|---|---|---|---|---|---|
| | A18 | B18 | C18 | a18 | b18 |
| Aqueous methanol solution (30-60) | 45 | 45 | 45 | 45 | 45 |
| EDTA (0.1-0.2) | 0.1 | 0.15 | 0.2 | 0.05 | 0.25 |
| Cholic acid (0.01-0.05) | 0.03 | 0.03 | 0.03 | 0.03 | 0.03 |
| 1 N aqueous acetic acid solution (0.05-0.1) | 0.07 | 0.07 | 0.07 | 0.07 | 0.07 |

Table 19 presents the experimental conditions in which the mixing ratio of only cholic acid is varied while the mixing ratios of other components are fixed in the redissolving process using the third solution for redissolution in the second embodiment of the present invention.

**[Table 19]**

| "Experiment 19" | Example | | | Comparative Example | |
|---|---|---|---|---|---|
| | A19 | B19 | C19 | a19 | b19 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.02 | 0.03 | 0.04 | 0.00 | 0.07 |
| 1 N aqueous acetic acid solution (0.05-0.1) | 0.07 | 0.07 | 0.07 | 0.07 | 0.07 |

Table 20 presents the experimental conditions in which the mixing ratio of only the 1 N aqueous acetic acid solution is varied while the mixing ratios of other components are fixed in the redissolving process using the third solution for redissolution in the second embodiment of the present invention.

**[Table 20]**

| "Experiment 20" | Example | | | Comparative Example | |
|---|---|---|---|---|---|
| | A20 | B20 | C20 | a20 | b20 |
| Aqueous methanol solution (30-60) | 45 | 45 | 45 | 45 | 45 |
| EDTA (0.1-0.2) | 0.15 | 0.15 | 0.15 | 0.15 | 0.15 |
| Cholic acid (0.01-0.05) | 0.03 | 0.03 | 0.03 | 0.03 | 0.03 |
| 1 N aqueous acetic acid solution (0.05-0.1) | 0.06 | 0.07 | 0.09 | 0.03 | 0.13 |

At this time, in Tables 17 to 20, in Examples (A17 to A20, B17 to B20, and C17 to C20), there was no particular problem in the process (for example, redissolution) of taking out the specimen from the vial 10. On the other hand, in Comparative Examples (a17 to a20 and b17 to b20), a problem (for example, specimen deformation) occurred in the process (for example, redissolution) of taking out the specimen from the vial 10.

Meanwhile, the solution for redissolution in the second embodiment of the present invention may be configured to contain 40 to 50 parts by weight of an aqueous ethanol solution, 0.1 to 0.2 parts by weight of EDTA, and 0.2 to 1 part by weight of a dihydric alcohol additive (hereinafter referred to as 'fourth solution for redissolution').

The physical properties depending on the composition ratio of such a fourth solution for redissolution were examined in the second embodiment of the present invention, and the influence of an individual component on the physical properties of the entire composition was examined while varying the mixing ratio of the individual component.

Table 21 presents the experimental conditions in which the mixing ratio of only the aqueous ethanol solution is varied while the mixing ratios of other components are fixed in the redissolving process using the fourth solution for redissolution in the second embodiment of the present invention.

**[Table 21]**

| "Experiment 21" | Example | | | Comparative Example | |
|---|---|---|---|---|---|
| | A21 | B21 | C21 | a21 | b21 |
| Aqueous ethanol solution (30-60) | 40 | 45 | 50 | 30 | 60 |
| EDTA (0.1-0.2) | 0.15 | 0.15 | 0.15 | 0.15 | 0.15 |
| Dihydric alcohol additive (0.02-1) | 0.06 | 0.06 | 0.06 | 0.06 | 0.06 |

Table 22 presents the experimental conditions in which the mixing ratio of only EDTA is varied while the mixing ratios of other components are fixed in the redissolving process using the fourth solution for redissolution in the second embodiment of the present invention.

**[Table 22]**

| "Experiment 22" | Example | | | Comparative Example | |
|---|---|---|---|---|---|
| | A22 | B22 | C22 | a22 | b22 |
| Aqueous ethanol solution (30-60) | 45 | 45 | 45 | 45 | 45 |
| EDTA (0.1-0.2) | 0.1 | 0.15 | 0.2 | 0.05 | 0.25 |
| Dihydric alcohol additive (0.02-1) | 0.06 | 0.06 | 0.06 | 0.06 | 0.06 |

Table 23 presents the experimental conditions in which the mixing ratio of only the dihydric alcohol additive is varied while the mixing ratios of other components are fixed in the redissolving process using the fourth solution for redissolution in the second embodiment of the present invention.

**[Table 23]**

| "Experiment 23" | Example | | | Comparative Example | |
|---|---|---|---|---|---|
| | A23 | B23 | C23 | a23 | b23 |
| Aqueous ethanol solution (30-60) | 45 | 45 | 45 | 45 | 45 |
| EDTA (0.1-0.2) | 0.15 | 0.15 | 0.15 | 0.15 | 0.15 |
| Dihydric alcohol additive (0.02-1) | 0.3 | 0.6 | 0.9 | 0.1 | 1.4 |

In Tables 21 to 23, in Examples (A21 to A23, B21 to B23, and C21 to C23), there was no particular problem in the process (for example, redissolution) of taking out the specimen from the vial 10. On the other hand, in Comparative Examples (a21 to a23 and b21 to b23), a problem (for example, specimen deformation) occurred in the process (for example, redissolution) of taking out the specimen from the vial 10.

## Claims

1. A cell examining method using jellification of an alcohol-based solution composition, the method comprising:
(a) mixing any one solution selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive with any one material selected from 5 to 20 parts by weight of one or more of gelatin or a photocurable protein, 1 to 100 parts by weight of a natural cellulose polymer material, or 1 to 100 parts by weight of synthesized acrylic polymer material beads at a temperature of 1°C to 40°C for jellification to produce an alcohol-based jelly-like material;
(b) putting a collected specimen inside an airtight container containing the alcohol-based jelly-like material;
(c) preparing a solution for redissolution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution;
(d) adding the jellified alcohol-based jelly-like material in the airtight container to the solution for redissolution;
(e) maintaining the solution for redissolution at a temperature of 1°C to 40°C to redissolve the alcohol-based jelly-like material into a liquid phase; and
(f) obtaining the specimen from the alcohol-based jelly-like material in a redissolved state and examining the specimen.

2. A cell examining method using jellification of an alcohol-based solution composition, the method comprising:
(a) putting any one solution (hereinafter referred to as 'selected solution') selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive into an airtight container;
(b) putting a specimen inside the airtight container filled with the selected solution;
(c) putting any one material selected from 5 to 20 parts by weight of one or more of gelatin or a photocurable protein, 1 to 100 parts by weight of a natural cellulose polymer material, or 1 to 100 parts by weight of synthesized acrylic polymer material beads inside the airtight container in a state of containing the selected solution and the specimen at a temperature of 1°C to 40°C and jellifying the mixture into an alcohol-based jelly-like material;
(d) preparing a solution for redissolution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution;
(e) adding the jellified alcohol-based jelly-like material in the airtight container to the solution for redissolution;
(f) maintaining the solution for redissolution at a temperature of 1°C to 40°C to redissolve the jellified alcohol-based jelly-like material into a liquid material; and
(g) obtaining the specimen from the liquid material in a redissolved state and examining the specimen.

3. A cell examining method using jellification of an alcohol-based solution composition, the method comprising:
(a) mixing any one solution selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive with any one material selected from 5 to 20 parts by weight of one or more of gelatin or a photocurable protein, 1 to 100 parts by weight of a natural cellulose polymer material, or 1 to 100 parts by weight of synthesized acrylic polymer material beads at a temperature of 1°C to 40°C for jellification to produce an alcohol-based jelly-like material;
(b) putting a collected specimen inside an airtight container containing the alcohol-based jelly-like material;
(c) preparing a solution for redissolution containing 40 to 50 parts by weight of an aqueous ethanol solution, 0.1 to 0.2 parts by weight of EDTA, and 0.2 to 1 part by weight of a dihydric alcohol additive;
(d) adding the jellified alcohol-based jelly-like material in the airtight container to the solution for redissolution;
(e) maintaining the solution for redissolution at a temperature of 1°C to 40°C to redissolve the alcohol-based jelly-like material into a liquid phase; and
(f) obtaining the specimen from the alcohol-based jelly-like material in a redissolved state and examining the specimen.

4. A cell examining method using jellification of an alcohol-based solution composition, the method comprising:
(a) putting any one solution (hereinafter referred to as 'selected solution') selected from a first solution containing 30 to 60 parts by weight of an aqueous methanol solution, 0.1 to 0.2 parts by weight of EDTA, 0.01 to 0.05 parts by weight of cholic acid, and 0.05 to 0.1 parts by weight of a 1 N aqueous acetic acid solution or a second solution containing 40 to 50 parts by weight of an aqueous ethanol solution, 0.1 to 0.2 parts by weight of EDTA, and 0.2 to 1 part by weight of a dihydric alcohol additive into an airtight container;
(b) putting a specimen inside the airtight container filled with the selected solution;
(c) putting any one material selected from 5 to 20 parts by weight of one or more of gelatin or a photocurable protein, 1 to 100 parts by weight of a natural cellulose polymer material, or 1 to 100 parts by weight of synthesized acrylic polymer material beads inside the airtight container in a state of containing the selected solution and the specimen at a temperature of 1°C to 40°C and jellifying the mixture into an alcohol-based jelly-like material;
(d) preparing a solution for redissolution containing 40 to 50 parts by weight of an aqueous ethanol solution, 0.1 to 0.2 parts by weight of EDTA, and 0.2 to 1 part by weight of a dihydric alcohol additive;
(e) adding the jellified alcohol-based jelly-like material in the airtight container to the solution for redissolution;
(f) maintaining the solution for redissolution at a temperature of 1°C to 40°C to redissolve the jellified alcohol-based jelly-like material into a liquid material; and
(g) obtaining the specimen from the liquid material in a redissolved state and examining the specimen.

## Patentansprüche

1. Zelluntersuchungsverfahren unter Verwendung von Gelierung einer alkoholbasierenden Lösungszusammensetzung, wobei das Verfahren aufweist:
(a) Mischen einer Lösung, die aus einer ersten Lösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0,1 Gew.-Teile einer wässrigen 1-N-Essigsäurelösung enthält, oder einer zweiten Lösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes mit einem Material, das aus 5 bis 20 Gew.-Teilen eines oder mehrerer Gelatine oder eines lichthärtbaren Proteins, 1 bis 100 Gew.-Teilen eines natürlichen Cellulosepolymermaterials oder 1 bis 100 Gew.-Teilen synthetisierter Acrylpolymermaterialperlen ausgewählt ist, ausgewählt ist, bei einer Temperatur von 1°C bis 40°C zur Gelierung, um ein alkoholbasierendes geleeartiges Material herzustellen;
(b) Einbringen einer gesammelten Probe innerhalb eines luftdichten Behälters, der das alkoholbasierende geleeartige Material enthält;
(c) Vorbereiten einer Lösung zur Wiederauflösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0,1 Gew.-Teile einer wässrigen 1-N-Essigsäurelösung enthält;
(d) Hinzufügen des gelierten alkoholbasierenden geleeartigen Materials in dem luftdichten Behälter zu der Lösung zur Wiederauflösung;
(e) Aufrechterhalten der Lösung zur Wiederauflösung bei einer Temperatur von 1°C bis 40°C, um das alkoholbasierende geleeartige Material in eine flüssige Phase aufzulösen; und
(f) Erhalten der Probe aus dem alkoholbasierenden geleeartigen Material in einem wiederaufgelösten Zustand und Untersuchen der Probe.

2. Zelluntersuchungsverfahren unter Verwendung von Gelierung einer alkoholbasierenden Lösungszusammensetzung, wobei das Verfahren aufweist:
(a) Einbringen einer Lösung (im Folgenden als "ausgewählte Lösung" bezeichnet), die aus einer ersten Lösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0,1 Gew.-Teilen einer wässrigen 1-N-Essigsäurelösung enthält, oder einer zweiten Lösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes enthält, ausgewählt ist, in einen luftdichten Behälter;
(b) Einbringen einer Probe innerhalb des mit der ausgewählten Lösung gefüllten luftdichten Behälters;
(c) Einbringen eines Materials, das aus 5 bis 20 Gew.-Teilen eines oder mehrerer Gelatine oder eines photohärtbaren Proteins, 1 bis 100 Gew.-Teilen eines natürlichen Cellulosepolymermaterials oder 1 bis 100 Gew.-Teilen synthetisierter Acrylpolymermaterialkügelchen ausgewählt ist, innerhalb des luftdichten Behälters in einem Zustand zum Enthalten der ausgewählten Lösung und der Probe bei einer Temperatur von 1°C bis 40°C, und Gelieren der Mischung zu einem alkoholbasierenden geleeartigen Material;
(d) Vorbereiten einer Lösung zur Wiederauflösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0,1 Gew.-Teile einer wässrigen 1-N-Essigsäurelösung enthält;
(e) Hinzufügen des gelierten alkoholbasierenden geleeartigen Materials in dem luftdichten Behälter zu der Lösung zur Wiederauflösung;
(f) Aufrechterhalten der Lösung zur Wiederauflösung bei einer Temperatur von 1°C bis 40°C, um das gelierte alkoholbasierende geleeartige Material in ein flüssiges Material aufzulösen; und
(g) Erhalten der Probe aus dem flüssigen Material in einem wiederaufgelösten Zustand und Untersuchen der Probe.

3. Zelluntersuchungsverfahren unter Verwendung von Gelierung einer alkoholbasierenden Lösungszusammensetzung, wobei das Verfahren aufweist:
(a) Mischen einer Lösung, die aus einer ersten Lösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0,1 Gew.-Teile einer wässrigen 1-N-Essigsäurelösung enthält, oder einer zweiten Lösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes mit einem Material enthält, das aus 5 bis 20 Gew.-Teilen eines oder mehrerer Gelatine oder eines photohärtbaren Proteins, 1 bis 100 Gew.-Teilen eines natürlichen Cellulosepolymermaterials oder 1 bis 100 Gew.-Teilen synthetisierter Acrylpolymermaterialperlen ausgewählt ist, ausgewählt ist, bei einer Temperatur von 1°C bis 40°C zur Gelierung, um ein alkoholbasierendes geleeartiges Material herzustellen;
(b) Einbringen einer gesammelten Probe innerhalb eines luftdichten Behälters, der das alkoholbasierende geleeartige Material enthält;
(c) Vorbereiten einer Lösung zur Wiederauflösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes enthält;
(d) Hinzufügen des gelierten alkoholbasierenden geleeartigen Materials in dem luftdichten Behälter zu der Lösung zur Wiederauflösung;
(e) Aufrechterhalten der Lösung zur Wiederauflösung bei einer Temperatur von 1°C bis 40°C, um das alkoholbasierende geleeartige Material in eine flüssige Phase aufzulösen; und
(f) Erhalten der Probe aus dem alkoholbasierenden geleeartigen Material in einem wiederaufgelösten Zustand und Untersuchen der Probe.

4. Zelluntersuchungsverfahren unter Verwendung von Gelierung einer alkoholbasierenden Lösungszusammensetzung, wobei das Verfahren aufweist:
(a) Hinzufügen einer Lösung (im Folgenden als "ausgewählte Lösung" bezeichnet), die aus einer ersten Lösung, die 30 bis 60 Gew.-Teile einer wässrigen Methanollösung, 0,1 bis 0,2 Gew.-Teile EDTA, 0,01 bis 0,05 Gew.-Teile Cholsäure und 0,05 bis 0. 1 Gew.-Teilen einer wässrigen 1-N-Essigsäurelösung enthält, oder einer zweiten Lösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes enthält, ausgewählt ist, in einen luftdichten Behälter;
(b) Einbringen einer Probe innerhalb des mit der ausgewählten Lösung gefüllten luftdichten Behälters;
(c) Einbringen eines Materials, das aus 5 bis 20 Gew.-Teilen eines oder mehrerer Gelatine oder eines photohärtbaren Proteins, 1 bis 100 Gew.-Teilen eines natürlichen Cellulosepolymermaterials oder 1 bis 100 Gew.-Teilen synthetisierter Acrylpolymermaterialkügelchen ausgewählt ist, innerhalb des luftdichten Behälters in einem Zustand vom Enthalten der ausgewählten Lösung und der Probe bei einer Temperatur von 1°C bis 40°C und Gelieren der Mischung zu einem alkoholbasierenden geleeartigen Material;
(d) Vorbereiten einer Lösung zur Wiederauflösung, die 40 bis 50 Gew.-Teile einer wässrigen Ethanollösung, 0,1 bis 0,2 Gew.-Teile EDTA und 0,2 bis 1 Gew.-Teil eines zweiwertigen Alkoholzusatzes enthält;
(e) Hinzufügen des gelierten alkoholbasierenden geleeartigen Materials in dem luftdichten Behälter zu der Lösung zur Wiederauflösung;
(f) Aufrechterhalten der Lösung zur Wiederauflösung bei einer Temperatur von 1°C bis 40°C, um das gelierte alkoholbasierende geleeartige Material in ein flüssiges Material wieder aufzulösen; und
(g) Erhalten der Probe aus dem flüssigen Material in einem wiederaufgelösten Zustand und Untersuchen der Probe.

## Revendications

1. Procédé d'examen de cellules utilisant la gélification d'une composition de solution à base d'alcool, le procédé comprenant :
(a) le mélange d'une solution quelconque sélectionnée parmi une première solution contenant 30 à 60 parties en poids d'une solution aqueuse de méthanol, 0,1 à 0,2 partie en poids d'EDTA, 0,01 à 0,05 partie en poids d'acide cholique et 0,05 à 0,1 partie en poids d'une solution aqueuse d'acide acétique 1 N ou une seconde solution contenant 40 à 50 parties en poids d'une solution aqueuse d'éthanol, 0,1 à 0,2 partie en poids d'EDTA et 0,2 à 1 partie en poids d'un additif d'alcool dihydrique avec une matière quelconque sélectionnée parmi 5 à 20 parties en poids d'une ou plusieurs parmi la gélatine ou une protéine photodurcissable, 1 à 100 parties en poids d'une matière polymère de cellulose naturelle, ou 1 à 100 parties en poids de billes de matière polymère acrylique synthétisée à une température de 1°C à 40°C pour la gélification afin de produire une matière de type gelée à base d'alcool ;
(b) le placement d'un spécimen collecté à l'intérieur d'un récipient hermétique contenant la matière de type gelée à base d'alcool ;
(c) la préparation d'une solution pour redissolution contenant 30 à 60 parties en poids d'une solution aqueuse de méthanol, 0,1 à 0,2 partie en poids d'EDTA, 0,01 à 0,05 partie en poids d'acide cholique et 0,05 à 0,1 partie en poids d'une solution aqueuse d'acide acétique 1 N ;
(d) l'ajout de la matière de type gelée à base d'alcool gélifiée dans le récipient hermétique à la solution pour redissolution ;
(e) le maintien de la solution pour redissolution à une température de 1°C à 40°C pour redissoudre la matière de type gelée à base d'alcool afin d'obtenir une phase liquide ; et
(f) l'obtention du spécimen à partir de la matière de type gelée à base d'alcool dans un état redissous et l'examen du spécimen.

2. Procédé d'examen de cellules utilisant la gélification d'une composition de solution à base d'alcool, le procédé comprenant :
(a) le placement d'une solution quelconque (ci-après dénommée 'solution sélectionnée') sélectionnée parmi une première solution contenant 30 à 60 parties en poids d'une solution aqueuse de méthanol, 0,1 à 0,2 partie en poids d'EDTA, 0,01 à 0,05 partie en poids d'acide cholique, et 0,05 à 0,1 partie en poids d'une solution aqueuse d'acide acétique 1 N ou une seconde solution contenant 40 à 50 parties en poids d'une solution aqueuse d'éthanol, 0,1 à 0,2 partie en poids d'EDTA et 0,2 à 1 partie en poids d'un additif d'alcool dihydrique dans un récipient hermétique ;
(b) le placement d'un spécimen à l'intérieur du récipient hermétique rempli de la solution sélectionnée ;
(c) le placement d'une matière quelconque sélectionnée parmi 5 à 20 parties en poids d'une ou plusieurs parmi la gélatine ou une protéine photodurcissable, 1 à 100 parties en poids d'une matière polymère de cellulose naturelle, ou 1 à 100 parties en poids de billes de matière polymère acrylique synthétisée à l'intérieur du récipient hermétique dans un état permettant de contenir la solution sélectionnée et le spécimen à une température de 1°C à 40°C et la gélification du mélange pour obtenir une matière de type gelée à base d'alcool ;
(d) la préparation d'une solution pour redissolution contenant 30 à 60 parties en poids d'une solution aqueuse de méthanol, 0,1 à 0,2 partie en poids d'EDTA, 0,01 à 0,05 partie en poids d'acide cholique et 0,05 à 0,1 partie en poids d'une solution aqueuse d'acide acétique 1 N ;
(e) l'ajout de la matière de type gelée à base d'alcool gélifiée dans le récipient hermétique à la solution pour redissolution ;
(f) le maintien de la solution pour redissolution à une température de 1°C à 40°C pour redissoudre la matière de type gelée à base d'alcool gélifiée afin d'obtenir une matière liquide ; et
(g) l'obtention du spécimen à partir de la matière liquide dans un état redissous et l'examen du spécimen.

3. Procédé d'examen de cellules utilisant la gélification d'une composition de solution à base d'alcool, le procédé comprenant :
(a) le mélange d'une solution quelconque sélectionnée parmi une première solution contenant 30 à 60 parties en poids d'une solution aqueuse de méthanol, 0,1 à 0,2 partie en poids d'EDTA, 0,01 à 0,05 partie en poids d'acide cholique et 0,05 à 0,1 partie en poids d'une solution aqueuse d'acide acétique 1 N ou une seconde solution contenant 40 à 50 parties en poids d'une solution aqueuse d'éthanol, 0,1 à 0,2 partie en poids d'EDTA et 0,2 à 1 partie en poids d'un additif d'alcool dihydrique avec une matière quelconque sélectionnée parmi 5 à 20 parties en poids d'une ou plusieurs parmi la gélatine ou une protéine photodurcissable, 1 à 100 parties en poids d'une matière polymère de cellulose naturelle, ou 1 à 100 parties en poids de billes de matière polymère acrylique synthétisée à une température de 1°C à 40°C pour la gélification afin de produire une matière de type gelée à base d'alcool ;
(b) le placement d'un spécimen collecté à l'intérieur d'un récipient hermétique contenant la matière de type gelée à base d'alcool ;
(c) la préparation d'une solution pour redissolution contenant 40 à 50 parties en poids d'une solution aqueuse d'éthanol, 0,1 à 0,2 partie en poids d'EDTA et 0,2 à 1 partie en poids d'un additif d'alcool dihydrique ;
(d) l'ajout de la matière de type gelée à base d'alcool gélifiée dans le récipient hermétique à la solution pour redissolution ;
(e) le maintien de la solution pour redissolution à une température de 1°C à 40°C pour redissoudre la matière de type gelée à base d'alcool afin d'obtenir une phase liquide ; et
(f) l'obtention du spécimen à partir de la matière de type gelée à base d'alcool dans un état redissous et l'examen du spécimen.

4. Procédé d'examen de cellules utilisant la gélification d'une composition de solution à base d'alcool, le procédé comprenant :
(a) le placement d'une solution quelconque (ci-après dénommée 'solution sélectionnée') sélectionnée parmi une première solution contenant 30 à 60 parties en poids d'une solution aqueuse de méthanol, 0,1 à 0,2 partie en poids d'EDTA, 0,01 à 0,05 partie en poids d'acide cholique, et 0,05 à 0,1 partie en poids d'une solution aqueuse d'acide acétique 1 N ou une seconde solution contenant 40 à 50 parties en poids d'une solution aqueuse d'éthanol, 0,1 à 0,2 partie en poids d'EDTA et 0,2 à 1 partie en poids d'un additif d'alcool dihydrique dans un récipient hermétique ;
(b) le placement d'un spécimen à l'intérieur du récipient hermétique rempli de la solution sélectionnée ;
(c) le placement d'une matière quelconque sélectionnée parmi 5 à 20 parties en poids d'une ou plusieurs parmi la gélatine ou une protéine photodurcissable, 1 à 100 parties en poids d'une matière polymère de cellulose naturelle, ou 1 à 100 parties en poids de billes de matière polymère acrylique synthétisée à l'intérieur du récipient hermétique dans un état permettant de contenir la solution sélectionnée et le spécimen à une température de 1°C à 40°C et la gélification du mélange pour obtenir une matière de type gelée à base d'alcool ;
(d) la préparation d'une solution pour redissolution contenant 40 à 50 parties en poids d'une solution aqueuse d'éthanol, 0,1 à 0,2 partie en poids d'EDTA et 0,2 à 1 partie en poids d'un additif d'alcool dihydrique ;
(e) l'ajout de la matière de type gelée à base d'alcool gélifiée dans le récipient hermétique à la solution pour redissolution ;
(f) le maintien de la solution pour redissolution à une température de 1°C à 40°C pour redissoudre la matière de type gelée à base d'alcool gélifiée afin d'obtenir une matière liquide ; et
(g) l'obtention du spécimen à partir de la matière liquide dans un état redissous et l'examen du spécimen.
